# EUROPÄISCHE PATENTSCHRIFT

(11) **EP 3 423 035 B1**
(45) Veröffentlichungstag und Bekanntmachung des Hinweises auf die Patenterteilung: **01.01.2020**
(21) Anmeldenummer: 18711043.2
(22) Anmeldetag: 05.03.2018
(51) Int. Cl.: A61K 9/08, A61K 47/02, A61K 38/46

(54) **WÄSSRIGE LÖSUNG VON BURLULIPASE UMFASSEND KALZIUMIONEN**
AQUEOUS SOLUTION OF BURLULIPASE COMPRISING CALCIUM IONS
COMPOSITION AQUEOUSE DE BURLULIPASE COMPRENANT DES IONS DE CALCIUM

(30) Priorität: 03.03.2017 DE 102017104480
(43) Veröffentlichungstag der Anmeldung: 09.01.2019
(73) Patentinhaber: Nordmark Arzneimittel GmbH & Co. KG, 25436 Uetersen (DE)
(72) Erfinder: FUHRHERR, Richard, 25436 Uetersen (DE); MÜLLER-LUCKS, Annika, 25436 Uetersen (DE); RÜTZEL, Katharina, 25436 Uetersen (DE); LÜDEMANN, Jan, 25436 Uetersen (DE); WERNER, Martin, 25436 Uetersen (DE)
(74) Vertreter: RGTH
(86) Internationale Anmeldenummer: PCT/EP2018/055380
(87) Internationale Veröffentlichungsnummer: WO 2018/158467

(56) Entgegenhaltungen:
- EP-A1- 3 090 638
- WO-A1-2010/085975
- DE-A1-102009 006 594
- DE-U1-202014 007 438

## Beschreibung

### Einleitung

Die vorliegende Erfindung betrifft eine wässrige Lösung umfassend Burlulipase, die dadurch gekennzeichnet ist, dass sie Kalziumionen enthält. Insbesondere betrifft sie auch entsprechende Arzneimittel. Solche Arzneimittel sind zur Behandlung von exokriner Pankreasinsuffizienz geeignet. Diese Arzneimittel sind insbesondere zur Behandlung von Patienten mit Mukoviszidose und in der Pädiatrie geeignet. Ein weiterer Aspekt der vorliegenden Erfindung betrifft in Behälter verpackte Lösungen und Arzneimittel, wie vorstehend genannt, wobei es sich um Behälter handelt, die mittels des Blow-Fill-Seal-Verfahrens hergestellt werden, und Verfahren zur Herstellung solcher verpackten Lösungen und Arzneimittel.

### Stand der Technik

Exokrine Pankreasinsuffizienz ist eine Krankheit, bei der die Bauchspeicheldrüse (auch das "Pankreas" genannt), keine oder nicht genügend Verdauungsenzyme bereitstellt. Zur Behandlung wird seit langem eine Enzymsubstitution angewendet, bei der ein aus den Bauchspeicheldrüsen von Schweinen gewonnenes Produkt, in Form von sogenanntem "Pankreatin" verabreicht wird. Dabei handelt es sich um einen Feststoff, der in einem mehrstufigen Verfahren hergestellt wird, das das Entfetten von Bauchspeicheldrüsen von Schweinen und das Entfernen von Fasern daraus umfasst. Pankreatin ist ein braunes Pulver, das neben den erwünschten Hauptbestandteilen Lipase, Protease und Amylase noch eine große Anzahl von Rückständen aus den Bauchspeicheldrüsen der Schweine enthält. Diese Behandlung ist sehr erfolgreich und meist ohne schwere Nebenwirkungen. Sie hat jedoch zwei Nachteile. Zum einen müssen große Mengen (große Massen) des Pankreatins eingenommen werden, was die Compliance beeinträchtigt. Zum anderen enthält das Pankreatin wechselnde Mengen an Verunreinigungen, die auch vom Rohstoff abhängen. Insbesondere enthält Pankreatin Restmengen an viralen und mikrobiellen Verunreinigungen, die aus dem Rohstoff stammen. Angesichts der Diskussion um die bovine spongiforme Enzephalopathie werden aus Säugetieren stammende biologische Verunreinigungen in Arzneimitteln von den Zulassungsbehörden immer kritischer gesehen. Zulassungen von Arzneimitteln, die aus tierischem oder menschlichem Gewebe hergestellt werden, sind daher zunehmend problematisch. Außerdem kann Pankreatin nicht in flüssiger Form verabreicht werden, was für die Verwendung in der Pädiatrie ein Hindernis darstellt.

In der EP 2 391 382 B1 werden pharmazeutische Präparate beschrieben, die Bakterien-Lipasen in wässrigen Lösungen enthalten. Diese sind für die Behandlung von Pankreasinsuffizienz, insbesondere bei Mukoviszidose und für die Behandlung von Kindern sowie für die Behandlung von Pankreatitis geeignet. Unter anderem wurde das Enzym Burlulipase aus *Burkholderia plantarii* (Triacylglycerollipase; EC-3.1.1.3) durch Expression gewonnen und seine Eignung zur Enzymsubstitution bei exokriner Pankreasinsuffizienz nachgewiesen. Burlulipase ist zur Therapie der exokrinen Pankreasinsuffizienz geeignet, kann jedoch nur unter bestimmten Bedingungen aufbewahrt und verabreicht werden. Insbesondere können Lösungen von Burlulipase nur gekühlt ohne Reduzierung der Aktivität für längere Zeit aufbewahrt werden. Es ist daher schwierig, Lösungen der Burlulipase zur Behandlung von exokriner Pankreasinsuffizienz bereitzustellen, die bei Raumtemperatur langzeitstabil sind und daher bei Raumtemperatur aufbewahrt werden können.

Es ist bekannt, dass verschiedene Reagenzien, wie Puffersysteme, Zucker, Metallionen, Emulgatoren usw. die Stabilität von Proteinen und insbesondere von Enzymen beeinflussen können. Für Pankreas-Lipase, wurde ein Einfluss von Kalziumionen auf die Aktivität nachgewiesen (siehe Kimura, H. et al, "Activiation of Human Pancreatic Lipase Activity by Calcium and Bile Salts", J. Biochem. 92, 243-251 (1982)) und für das menschliche Wachstumshormon wurde ein Einfluss von Kalziumionen auf die Stabilität nachgewiesen (siehe Saboury, A. A. et al, "Effect of calcium binding on the structure and stability of human growth hormone", International Journal of Biological Macromolecules 36 (2005) 305-309).

In dem Artikel von Ahmed M.K. Youssef und Gerhard Winter, "A critical evaluation of microcalorimetry as a predictive tool for long term stability of liquid protein formulations: Granulocyte Colony Stimulating Factor (GCSF)", European Journal of Pharmaceutics and Biopharmaceutics 84 (2013), Seiten 145 bis 155, wird Micro-Differential-Scanning-Calorimetry (dynamische Mikro-Differenzkalorimetrie) als Methode zur Bestimmung von Änderungen der Tertiärstruktur von Proteinen, insbesondere von Enzymen, in Lösungen etabliert. Diese Methode ist daher geeignet, die Stabilität von Enzymen in Lösungen zu bestimmen. Aus diesem Grund wird diese analytische Methode in der vorliegenden Anmeldung verwendet. Im Folgenden wird Micro-Differential-Scanning-Calorimetry auch als Mikrokalorimetrie bezeichnet oder mit µDSC abgekürzt.

Die WO 2010/085975 A1 betrifft pharmazeutische Präparate zur Behandlung von Pankreasinsuffizienz, beispielsweise Mukoviszidose oder anderer Pankreaserkrankungen. Insbesondere betrifft die vorliegende Erfindung flüssige pharmazeutische Präparate von Verdauungsenzymen, die keinen Überzug haben und in der aggressiven Umgebung von Magen/Zwölffingerdarm aktiv sind. Außerdem sind diese flüssigen pharmazeutischen Präparate stabil und können bei hohen Temperaturen gelagert werden. Bei der flüssigen Formulierung kann es sich auch um Lösungen und Suspensionen handeln.

### Aufgabe der vorliegenden Erfindung

Es war eine Aufgabe der vorliegenden Erfindung, Lösungen der Burlulipase bereitzustellen, die gegenüber Lösungen von der Burlulipase in reinem Wasser eine erhöhte thermische Stabilität aufweisen. Insbesondere sollen die Lösungen bei Temperaturen von mehr als 10 °C eine höhere Lagerfähigkeit aufweisen, und ganz besonders bevorzugt sollen die Lösungen bei 15 bis 35 °C eine höhere Lagerfähigkeit aufweisen. Insbesondere ist es auch eine Aufgabe der vorliegenden Erfindung, Burlulipase-Zubereitungen bereitzustellen, die eine genügende thermische Stabilität aufweisen, die es erlaubt, sie auch nach sequenzieller Lagerung einzunehmen. Ein weiteres Ziel der vorliegenden Erfindung ist es, Zubereitungen der Burlulipase bereitzustellen, die möglichst leicht herstellbar und möglichst steril sind, die genau dosierbar sind und leicht eingenommen werden können.

### Beschreibung der Erfindung

Der Internationale Freiname (International Nonproprietary Name; INN) als gemeinfreier Name für eine arzneiliche Wirksubstanz wird von der Weltgesundheitsorganisation (World Health Organization; WHO) vergeben. Als solcher bezieht sich der INN-Name Burlulipase zunächst ausschließlich auf das lipolytisch aktive Protein als reine Wirksubstanz. In der realen Produktion pharmazeutischer Wirkstoffe stellt dieser Reinstzustand allerdings ein theoretisches Ideal dar, da die Wirkstoffe, ggf. auch nur in Spuren, so doch immer Begleitsubstanzen und Verunreinigungen enthalten. Auch der Wirkstoff Burlulipase enthält damit neben der Wirksubstanz Burlulipase (INN) unvermeidbar Begleitstoffe und Verunreinigungen. Im Sinne der vorliegenden Erfindung sollen daher unter dem Begriff "Burlulipase" die unterschiedlichen Qualitäten dieses Wirkstoffs verstanden werden. Insbesondere fällt der Wirkstoff Burlulipase während des Herstellungsverfahrens zunächst in einer Qualität an, die noch erhebliche Mengen des Kohlenhydrats 6-Desoxy-Talan als Begleitsubstanz enthält, eines Gemisches aus Polymeren und Oligomeren der 6-Desoxy-Talose (siehe Zähringer, U. et al., "Structure of a new 6-deoxy-α-D-talan from Burkholderia (Pseudomonas) plantarii strain DSM 6535, which is different from the O-chain of the lipopolysaccharide", Carbohydrate Research 300 (1997), 143-151. Analytisch detektiert wird 6-Desoxy-Talan i. d. R. in Form seines Monomers 6-Desoxy-Talose. Durch weitreichende Abtrennung des 6-Desoxy-Talan kann daraus aber auch eine sehr reine Qualität des Wirkstoffs Burlulipase gewonnen werden, der nur noch geringe Mengen 6-Desoxy-Talan enthält. Beide Qualitäten enthalten über 6-Desoxy-Talan hinaus geringe Mengen weiterer Begleitstoffe und Verunreinigungen, darunter als Lipopolysaccharide insbesondere Rhamnolipide, des weiteren aber u. a. auch Rhamnose und Lipide. Im Sinne der vorliegenden Erfindung sollen daher unter dem Begriff "Burlulipase" sämtliche Qualitäten dieses Wirkstoffs, insbesondere auch die beiden voranstehend beschriebenen Qualitäten verstanden werden. Die vorliegende Erfindung betrifft eine Zubereitung von Burlulipase, bei der es sich um eine wässrige Lösung umfassend Burlulipase handelt, die dadurch gekennzeichnet ist, dass sie Kalziumionen enthält. Kalziumionen haben die Eigenschaft, dass sie Lösungen von Burlulipase in Wasser gegen thermische Belastungen stabilisieren. Wässrige Lösungen, die Burlulipase und Kalziumionen enthalten, können daher länger oder bei höheren Temperaturen gelagert werden, ohne dass sich im Vergleich zu einer Lösung, die keine Kalziumionen enthält, der Aktivitätsverlust erhöht. Dadurch wird das Haltbarkeitsdatum verlängert oder die Lagertemperatur kann erhöht werden. Bevorzugt sind dabei hierin beschriebene wässrige Lösungen, die Kalziumchlorid enthalten. Dabei ist es unwesentlich, ob das Kalziumchlorid als solches zugegeben wurde, oder ob die Kalziumionen und die Chloridionen aus verschiedenen Quellen stammen, so lange die Lösung Kalziumchlorid, das heißt sowohl Kalziumionen als auch Chloridionen, enthält. Kalziumchlorid ist eine physiologisch verträgliche Form der Kalziumionen, die den Effekt der Kalziumionen auf die Burlulipase nicht beeinträchtigt.

Die vorliegenden wässrigen Lösungen, wie hierin beschrieben, beinhalten bevorzugt wenigstens 10 Gew.-% Wasser, besonders bevorzugt wenigstens 30 Gew.-% Wasser und noch mehr bevorzugt wenigstens 40 Gew.-% Wasser, ganz besonders bevorzugt wenigstens 50 Gew.-% Wasser und am meisten bevorzugt wenigstens 70 Gew.-% Wasser. Es ist ebenfalls bevorzugt, dass die erfindungsgemäßen wässrigen Lösungen weniger als 30 Gew.-%, bevorzugt weniger als 10 Gew.-%, ganz besonders bevorzugt 5 % und am meisten bevorzugt weniger als 1 Gew.-% Alkohole mit einer Kohlenstoffzahl von 6 oder weniger enthalten.

Die Burlulipase liegt in den erfindungsgemäßen Lösungen in der Regel in einer Massenkonzentration von 0,1 bis 35 mg Protein/ml vor. Insofern im Folgenden für Konzentrationen die Einheit einer Masse pro Volumen wie mg/ml verwendet wird, handelt es sich jeweils um Massenkonzentrationen. Für besondere Anwendungen können jedoch auch Massenkonzentrationen außerhalb dieses Bereichs verwendet werden. Bevorzugt liegt die Burlulipase in einer Konzentration von 0,5 bis 30 mg Protein/ml, ganz besonders bevorzugt in einer Konzentration von 1 bis 25 mg Protein/ml und am meisten bevorzugt in einer Konzentration von 3 bis 20 mg Protein/ml, vor. Eine Konzentration von 0,1 bis 35 mg Protein/ml ist leicht herstellbar und in der Regel physiologisch aktiv genug, um in der Behandlung eines Patienten eingesetzt werden zu können. Eine Konzentration von 0,5 bis 30 mg Protein/ml ist bevorzugt, weil oft mindestens eine Konzentration von 0,5 mg Protein/ml notwendig ist, um eine genügende Dosierung zu erreichen, und eine Konzentration von 30 mg Protein/ml in einer geeigneten Formulierung auch bei Raumtemperatur über einen Zeitraum von mindestens 6 Monaten stabil ist. Eine Konzentration von 1 bis 25 mg Protein/ml ist für die meisten Therapien ausreichend, und in aller Regel wird eine Konzentration von 3 bis 20 mg Protein/ml angemessen sein. Für die Anwendung in der Pädiatrie sind Konzentrationen von 2 bis 10 mg Protein/ml bevorzugt.

Die erfindungsgemäße wässrige Lösung, wie sie hierin beschrieben wird, kann im Prinzip beliebige Mengen an Kalziumionen enthalten. Bereits in sehr kleinen Mengen zeigen Kalziumionen einen stabilisierenden Effekt. Die obere Grenze wird im Wesentlichen durch die physiologische Verträglichkeit von Kalziumionen bestimmt. In der Regel sind jedoch Kalziumionen in einer Konzentration von bis zu 200 mmol/l enthalten, bevorzugt in einer Konzentration von 0,01 bis 150 mmol/l. Soweit im Folgenden für Konzentrationen die Einheit einer Stoffmenge pro Volumen, wie mmol/l verwendet wird, handelt es sich jeweils um Stoffmengenkonzentrationen. Kalziumionen in dieser Menge sind in genügender Menge vorhanden, um im Wesentlichen alle Burlulipase-Moleküle zu stabilisieren, sind aber gleichzeitig nicht so hoch konzentriert, dass sie zu Nebenwirkungen führen. Dies gilt insbesondere für den Bereich von 0,1 bis 50 mmol/l, der daher besonders bevorzugt ist. Ganz besonders bevorzugt ist eine erfindungsgemäße wässrige Lösung, wie sie hierin beschrieben ist, die Kalziumionen in einer Menge von 0,5 bis 20 mmol/l enthält. Am meisten bevorzugt ist jedoch eine Menge von 5 bis 30 mmol. Diese geringen Mengen stellen zusätzlich sicher, dass die physiologisch verträgliche Menge an Kalziumchlorid nicht überschritten wird.

Insbesondere bevorzugt ist eine erfindungsgemäße wässrige Lösung, wie hierin beschrieben, die Kalziumchlorid in den vorstehend für Kalziumionen angegebenen Stoffmengenkonzentrationen enthält. Die Gründe hierfür sind wie vorstehend für die Verwendung von Kalziumchlorid und die Mengen der Kalziumionen beschrieben.

Wässrige Lösungen von Burlulipase sind in der Regel am stabilsten in einem pH-Bereich von 4 bis 9. Daher sind erfindungsgemäße wässrige Lösungen, wie hierin beschrieben bevorzugt, die einen pH-Wert von 4 bis 9 aufweisen. Mehr bevorzugt sind solche erfindungsgemäßen wässrigen Lösungen, die einen pH-Wert von 6 bis 8 und besonders bevorzugt von 7 bis 8 aufweisen. Am stabilsten sind die erfindungsgemäßen wässrigen Lösungen in einem pH-Bereich von 7,3 bis 7,7 und solche erfindungsgemäßen Lösungen sind daher am meisten bevorzugt, die einen pH-Wert von 7,3 bis 7,7 aufweisen.

Zur Einstellung des pH-Wertes können den erfindungsgemäßen wässrigen Lösungen, wie sie hierin beschrieben werden, in einfacher Weise Säuren und Basen zugegeben werden. Die Wahl der Säuren und Basen ist hierbei nicht besonders beschränkt. Alle möglichen Säuren und Basen können hierzu verwendet werden. Säuren mit niedrigem pKs-Wert wie Salzsäure oder Schwefelsäure und Basen mit hohem pKs-Wert, wie zum Beispiel Alkalihydroxide sind besonders geeignet. Ebenfalls bevorzugt werden pH-Puffer verwendet, um den pH-Wert geeignet einzustellen. Solche pH-Puffer gewährleisten, dass der pH-Wert dauerhaft erhalten bleibt, und verhindern, dass Schwankungen der Säure- und Basengehalte der zur Herstellung der erfindungsgemäßen Lösungen verwendeten Rohstoffe Einfluss auf den pH-Wert der erfindungsgemäßen Lösungen haben.

Bevorzugt wird Tris(hydroxymethyl)aminomethan als Bestandteil des pH-Puffers in den erfindungsgemäßen wässrigen Lösungen eingesetzt. Bevorzugt ist daher eine wässrige Lösung gemäß einem der vorangehenden Ansprüche, die Tris(hydroxymethyl)aminomethan enthält.

Des Weiteren können die erfindungsgemäßen wässrigen Lösungen pharmazeutisch akzeptable Hilfsstoffe in üblichen Mengen enthalten, die dem Fachmann aus dem Stand der Technik bekannt sind. Insbesondere können die erfindungsgemäßen wässrigen Lösungen Emulgatoren, Lösungshilfsmittel, Geschmacks-, Geruchs-, und Farbstoffe, Lösungsmittel, Zucker (Glucose, Laktose), bevorzugt nicht-reduzierende Zucker (Trehalose, Saccharose), Alkohole, Zuckeralkohole (z.B. Mannitol, Sorbitol), Lipide, Aminosäuren, Salze, Elektrolyte, Antioxidantien, Konservierungsstoffe und antimikrobiotisch wirksame Substanzen enthalten. Weitere geeignete Hilfsstoffe können zum Beispiel dem Lexikon der Hilfsstoffe für Pharmazie, Kosmetik und angrenzende Gebiete (Fiedler, Herbert P.; Bd.2, 40 OVR Oberschwäbische Verlagsanstalt Ravensburg, 1989) entnommen werden. Die erfindungsgemäße wässrige Lösung kann ebenfalls Trägerstoffe enthalten, wobei Polyole und insbesondere Monosaccharide und Polysaccharide bevorzugt sind. Besonders geeignete Hilfsstoffe sind z. B. im Buch "Pharmaceutical Formulation Development of Peptides and Proteins" Second Edition (CRC Press, Autoren: Lars Hovgaard, Sven Frokjaer, Marco van de Weer) u. a. in den Kapiteln 8 und 10 aufgeführt. Weitere geeignete Hilfsstoffe sowie Kombinationen davon finden sich in zahlreichen Veröffentlichungen und Patentanmeldungen von Wolfgang Frieß, Gerhard Winter, John Carpenter, Michael Pikal, Patrick Garidel und Hanns-Christian Mahler.

Wie oben bereits beschrieben kann die erfindungsgemäße wässrige Lösung einer entsprechenden Qualität des Wirkstoffs Burlulipase, wie hierin beschrieben, erhebliche Mengen an 6-Desoxy-Talan als Begleitsubstanz enthalten. Besonders bevorzugt enthält sie in diesen Fällen 6-Desoxy-Talan in einer Menge von 1 bis 60 Gew.-% bezogen auf den getrockneten Wirkstoff und am meisten bevorzugt enthält sie in diesen Fällen 6-Desoxy-Talan in einer Menge von 15 bis 40 Gew.-% bezogen auf die getrocknete Burlulipase. 6-Desoxy-Talan trägt jedoch auch zur thermischen Instabilität von Burlulipase bei. Daher sind erfindungsgemäße wässrige Lösungen bevorzugt, in denen 6-Desoxy-Talan in einer Menge von 10 Gew.-% oder weniger, besonders bevorzugt von 2 Gew.-% oder weniger und ganz besonders bevorzugt von 1 Gew.-% oder weniger enthalten sind, jeweils bezogen auf die Burlulipase.

Weiterhin kann die erfindungsgemäße wässrige Lösung, wie hierin beschrieben, Verunreinigungen enthalten, die aus dem Herstellungsverfahren stammen. Burlulipase wird in der Regel durch Expression aus *Burkholderia plantarii* gewonnen. Bei der Aufreinigung können die meisten Verunreinigungen entfernt werden. Ein kleiner Teil kann jedoch auch nach Aufreinigung in dem Produkt enthalten sein.

Ein weiterer Aspekt der vorliegenden Erfindung ist ein Arzneimittel umfassend eine erfindungsgemäße wässrige Lösung, wie hierin beschrieben. Der große Vorteil, den die erfindungsgemäße wässrige Lösung, wie hierin beschrieben, gegenüber aus Tieren gewonnenen Lipasen hat, ist, dass sie keine viralen oder mikrobiellen Verunreinigungen aufweist. Insbesondere enthält sie keine Viren, Bakterien oder Prionen aus tierischen Quellen und ist daher in dieser Hinsicht als ungefährlich und nebenwirkungsfrei einzustufen.

Bevorzugt ist das erfindungsgemäße Arzneimittel, wie hierin beschrieben, ein Arzneimittel zur Behandlung von exokriner Pankreasinsuffizienz. Die Wirkung von Burlulipase bei der Behandlung von exokriner Pankreasinsuffizienz ist bereits bekannt und das erfindungsgemäße Arzneimittel kann in dieser Therapie das üblicherweise verwendete Pankreatin ablösen. Das erfindungsgemäße Arzneimittel hat dabei gegenüber Pankreatin den Vorteil, dass es keine viralen oder mikrobiellen Verunreinigungen enthält. Insbesondere geeignet ist das erfindungsgemäße Arzneimittel, wie hierin beschrieben, zur Behandlung von Pankreasinsuffizienz bei Patienten mit Mukoviszidose. Ferner ist das erfindungsgemäße Arzneimittel, wie hierin beschrieben, zur Anwendung in der Pädiatrie geeignet. Pankreatin kann nur als Feststoff zur Verfügung gestellt werden und muss täglich in großen Mengen, großen Darreichungsformen und/oder in einer großen Anzahl von Darreichungsformen (z.B. Tabletten oder Kapseln) eingenommen werden. Dies ist gerade für Kinder oft schwierig. Die vorliegenden wässrigen Lösungen können als Getränke oder in Getränken in Begleitung von Mahlzeiten verabreicht werden, sind daher auch für Kinder leicht zugänglich und ermöglichen auch in der Pädiatrie eine hohe Compliance.

Besonders bevorzugt ist das erfindungsgemäße Arzneimittel, wie hierin beschrieben, zur Anwendung nach sequenzieller Lagerung vorgesehen und geeignet. Unter sequenzieller Lagerung im Sinne dieser Anmeldung wird verstanden, dass das Arzneimittel zunächst in einer ununterbrochenen Kühlkette gelagert wird, jedoch in der Zeit unmittelbar vor sowie in der Zeit nach Anbruch einer Packung diese Packung bei Raumtemperatur aufgehoben werden kann, bis sie aufgebraucht worden ist. Dies ist wichtig, um eine genügende Versorgung der Patienten mit dem Wirkstoff Burlulipase zu gewährleisten. Ohne Stabilisierung durch Kalziumionen ist die Stabilität der Burlulipase begrenzt. Auch wenn eine ununterbrochene Kühlkette beim Hersteller, im Großhandel und in Apotheken vorhanden ist, fällt es dem Patienten oft schwer, eine dauerhafte Kühlung sicherzustellen. Abgesehen davon, dass viele Patienten, besonders in ärmeren Ländern, keine Kühlmöglichkeit besitzen, fällt es Patienten oft schwer, die notwendige Disziplin aufzubringen, die Kühlung zu gewährleisten. Insbesondere auf Reisen oder bei ganztägiger Abwesenheit von Zuhause ist dies häufig ein Problem. Dies führt gegebenenfalls zu einer Unterversorgung der Patienten. Dies gilt insbesondere, da der Patient keine Möglichkeit hat, die verbleibende Aktivität der Burlulipase zu bestimmen.

Das erfindungsgemäße Arzneimittel enthält jedoch eine stabilisierte Form der Burlulipase, die es erlaubt, das Arzneimittel auch über längere Zeit außerhalb eines gekühlten Ortes aufzubewahren, ohne dass die Aktivität des Wirkstoff unter die für die Wirksamkeit erforderliche Aktivität sinkt. Je nach Witterung kann das erfindungsgemäße Arzneimittel bis zu vier Wochen oder länger bei Umgebungstemperatur aufbewahrt werden, ohne dass die Aktivität zu sehr abnimmt. Je nach Klimazone kann die Packungsgröße so angepasst werden, dass diese bei passender Dosierung aufgebraucht ist, bevor die Aktivität der Lipase durch thermische Deaktivierung zu stark gesunken ist. Das erfindungsgemäße Arzneimittel kann daher gefahrlos vom Patienten selbst mitgeführt und eingenommen werden, ohne das die Gefahr einer falschen Dosierung besteht.

Ein weiterer Aspekt der vorliegenden Erfindung ist eine erfindungsgemäße wässrige Lösung, wie hierin beschrieben, oder ein erfindungsgemäßes Arzneimittel, wie hierin beschrieben, worin die Lösung oder das Arzneimittel in einem Behälter verpackt ist, der nach dem Blow-Fill-Seal-Verfahren hergestellt wurde.

Ebenfalls ein weiterer Aspekt der vorliegenden Erfindung ist ein Verfahren zur Herstellung einer verpackten wässrigen Lösung, wie hierin beschrieben, oder eines verpackten erfindungsgemäßen Arzneimittels, wie hierin beschrieben, worin die erfindungsgemäße wässrige Lösung oder das erfindungsgemäße Arzneimittel nach dem Blow-Fill-Seal-Verfahren verpackt wird. Insbesondere bevorzugt ist dabei ein Verfahren, wie hierin beschrieben, umfassend die Schritte:
- Bereitstellen eines Polymers,
- Extrudieren des Polymers durch eine ringförmige horizontale Öffnung, so dass ein hängender Schlauch entsteht,
- Abschneiden des Schlauchs,
- Schließen des Schlauchs am unteren Ende,
- Einbringen des Schlauchs in eine Form,
- Aufblasen des Schlauchs, so dass der Schlauch die Form ausfüllt und das Material des Schlauchs die Wandung eines Behälters formt,
- Einfüllen einer erfindungsgemäßen Lösung oder eines erfindungsgemäßen Arzneimittels in den Behälter,
- Schließen des Behälters,
wobei zumindest das Einfüllen der Lösung bzw. des Arzneimittels und das Schließen des Containers unter sterilen Bedingungen erfolgen. Die Schritte des Abschneidens des Schlauchs, des Schließens des Schlauchs am unteren Ende und des Einbringens des Schlauchs in eine Form können dabei in beliebiger Reihenfolge zueinander erfolgen. Alle anderen Verfahrensschritte erfolgen bevorzugt in der aufgeführten Reihenfolge. Besonders bevorzugt erfolgen alle Verfahrensschritte in der vorstehend angeführten Reihenfolge.

Behälter, die nach den Blow-Fill-Seal-Verfahren hergestellt werden, sind Plastikbehälter, die durch einfaches Aufreißen oder Aufschneiden oder dergleichen geöffnet werden. Damit ist das Arzneimittel oder die Lösung im Gegensatz zu Glasbehältern ohne Aufwand verfügbar und auch für Laien, insbesondere Patienten, einfach handhabbar. So ist etwa anders als bei Phiolen das Hantieren mit Nadeln und dergleichen nicht notwendig. Glasbruch kann ebenfalls nicht auftreten. Im Vergleich zu Glasflaschen haben sie den Vorteil, dass die Arzneimittel portionsweise abgefüllt sind und eine genaue Dosierung erlauben. Behälter, die nach den Blow-Fill-Seal-Verfahren hergestellt werden, sind daher ideal geeignet zur Verabreichung von flüssigen Arzneimitteln zur Einnahme durch die Patienten selbst. Dies gilt insbesondere für Indikationen in der Pädiatrie.

### Beispiele:

Burlulipase wurde gemäß dem Beispiel 1 der EP 0 592 478 B2 hergestellt. Dies gilt für alle Versuche.

### Beispiel 1: Voruntersuchungen zur Stabilisierung von Burlulipase

Um die Wirkung von verschiedenen Zusätzen auf die thermische Stabilität von wässrigen Lösungen der Burlulipase zu studieren, wurden verschiedene Lösungen von Burlulipase in Wasser mittels Mikrokalorimetrie vermessen. Die Mikrokalorimetrie ist eine analytische Methode bei der die Burlulipase (oder ein anderes Protein) in der entsprechenden Formulierung erhitzt wird. Dabei wird die Temperatur bei der die Entfaltung des Proteins stattfindet, detektiert. Es ist nachgewiesen, dass eine höhere Entfaltungstemperatur häufig mit einer erhöhten thermischen Stabilität des Proteins einhergeht. Diese wiederum kann eine längere Laufzeit oder eine höhere Lagertemperatur bedingen. Details zu dieser analytischen Methode finden sich in dem Artikel von Youssef und Winter der vorstehend genannt ist.

Es wurde ein dynamisches Differenzkalorimeter Typ Nano DSC der Fa. TA Instruments mit Hauptsitz in New Castle (Delaware, USA) verwendet. Gemessen wurde mit einem Temperaturanstieg von 1 K/min. Es wurde eine Lösung von 2 mg/ml Burlulipase in Wasser hergestellt. Eine weitere Lösung die 2 mg/ml Burlulipase und 10 mmol/l Kalziumchlorid in Wasser enthält wurde ebenfalls hergestellt. Die Lösungen enthielten keine weiteren Bestandteile. Abbildungen 1 und 2 zeigen die mikrokalorimetrischen Diagramme dieser beiden Lösungen. Abbildung 1 zeigt, einen Peak bei etwa 73 °C. Die kleine Kurve ist eine berechnete Kurve, die einen Nebenpeak similiert und keine Wiedergabe der eigentlichen Messdaten. Diese Abbildung zeigt, dass sich Burlulipase in wässriger Lösung bei etwa 73 °C entfaltet. Abbildung 2 zeigt die entsprechende Kurve für die Kalziumchlorid-haltige Lösung. Hier liegt die Entfaltungstemperatur bei etwa 88 °C, also um etwa 15 °C höher. Dies ist ein klarer Hinweis, dass Kalziumchlorid Burlulipase stabilisieren könnte.

### Beispiel 2: Messung der lipolytischen Aktivität von wässrigen Lösungen der Burlulipase

Wässrige Lösungen der Burlulipase wurden durch Auflösen der Bestandteile in Wasser hergestellt. Es wurden 4 Lösungen hergestellt. Der pH-Wert betrug bei allen Lösungen 7,5. Tabelle 1 gibt die Zusammensetzungen der 4 Lösungen wieder:

**Tabelle 1:**

| Zutaten | Lösung 1 | Lösung 2 | Lösung 3 | Lösung 4 |
|---|---|---|---|---|
| Burlulipase [mg/ml] | 15 | 15 | 15 | 15 |
| Calciumchlorid [mmol] | - | 1 | 10 | - |
| NaH₂PO₄ [mmol] | - | - | - | 5 |
| Wasser | Rest | Rest | Rest | Rest |

Die 4 Lösungen wurden bei vier verschiedenen Temperaturen für einen Zeitraum von mehreren Monaten unter sonst identischen Bedingungen aufbewahrt. In bestimmten Intervallen wurde die Aktivität der Burlulipase gemessen. Die folgenden Tabellen geben die Ergebnisse der Versuche wieder. Alle Aktivitäten sind in Prozent der bei Herstellung der Lösungen gemessenen lipolytischen Aktivität angegeben.

**Tabelle 2: Temperatur 2 bis 8 °C**

| Lösung Nr. | Beginn | 3 Monate | 6 Monate | 9 Monate | 12 Monate | 15 Monate |
|---|---|---|---|---|---|---|
| 1 | 100 | 107,7 | 102,7 | 108,7 | 96,8 | 107,6 |
| 2 | 100 | 107,7 | 105,4 | 104,1 | 100,6 | 114,8 |
| 3 | 100 | 111,4 | 109,1 | 113,2 | 108,7 | 109,1 |
| 4 | 100 | 104,2 | 97,4 | 96,0 | 95,8 | 104,7 |

**Tabelle 3: Temperatur 25 °C**

| Lösung Nr. | Beginn | 3 Monate | 6 Monate | 9 Monate | 12 Monate |
|---|---|---|---|---|---|
| 1 | 100 | 94,8 | 84,6 | 85,9 | 81,3 |
| 2 | 100 | 109,4 | 102,8 | 101,7 | 79,5 |
| 3 | 100 | 113,1 | 106,4 | 110,0 | 107,7 |
| 4 | 100 | 92,2 | 83 | 80,2 | 76,1 |

**Tabelle 4: Temperatur 30 °C**

| Lösung Nr. | Beginn | 3 Monate | 6 Monate | 9 Monate | 12 Monate |
|---|---|---|---|---|---|
| 1 | 100 | 95,1 | 83,2 | 82,6 | 82,2 |
| 2 | 100 | 113,6 | 99,6 | 105,4 | 102,0 |
| 3 | 100 | 120,0 | 102,5 | 112,7 | 110,4 |
| 4 | 100 | 104,3 | 85,1 | 86,8 | 82,5 |

**Tabelle 5: Temperatur 40 °C**

| Lösung Nr. | Beginn | 3 Monate | 6 Monate | 9 Monate | 12 Monate |
|---|---|---|---|---|---|
| 1 | 100 | 62,3 | 47,0 | nicht gemessen | |
| 2 | 100 | 102,7 | 84,1 | nicht gemessen | |
| 3 | 100 | 105,5 | 95,2 | nicht gemessen | |
| 4 | 100 | 55,2 | 41,8 | nicht gemessen | |

Die Daten zeigen, dass Kalziumionen die Burlulipase stabilisieren können. Bei einer Konzentration von 10 mmol (Lösung 3) ist der Effekt deutlich ausgeprägter als bei einer Konzentration von 1 mmol (Lösung 4). Temperaturen von 25 bis 40 °C entsprechen den Temperaturen denen ungekühlte Arzneistoffe ausgesetzt sind. Die vorliegende Erfindung ermöglicht es daher, flüssige Arzneimittel herzustellen die Burlulipase enthalten. Erfindungsgemäße Arzneimittel sind gegenüber nicht mit Kalziumionen stabilisieren Arzneimittel stabil genug um eine stabile Versorgung der Patienten mit Burlulipase zu gewährleisten. Phosphat scheint hingegen einen sehr leicht destabilisierenden Effekt auf Burlulipase zu haben. In jedem Fall sind die erfindungsgemäßen durch Kalziumionen stabilisierten wässrige Lösungen für die sequenzielle Lagerung geeignet.

### Beispiel 3: Effekt des 6-Desoxy-Talans

In diesem Versuch wurden zwei Burlulipasen mit verschiedenen 6-Desoxy-Talan-Gehalten verglichen. Eine Abreicherung des in der Burlulipase enthaltenen 6-Desoxy-Talan kann durch eine Größenausschlusschromatographie (Size Exclusion Chromatography, SEC) erzielt werden. Auch andere Verfahren sind hierfür bekannt.

Die folgende Tabelle zeigt Untersuchungen zur thermischen Stabilität von wässrigen Lösungen von Burlulipase, die 15 mg/ml Burlulipase enthalten. Dabei enthält eine Art der Burlulipase 30 Gew.-% 6-Desoxy-Talan bezogen auf die getrocknete Burlulipase und eine zweite Lösung enthält 1 Gew.-% 6-Desoxy-Talan bezogen auf die getrocknete Burlulipase. Der pH-Wert der Lösungen betrug zu Beginn der Messungen 7,5. Die Messungen wurden bei zwei verschiedenen Temperaturen (25 °C und 40 °C) durchgeführt. Die Lösungen enthielten außer der Burlulipase keine weiteren Zutaten. Die Lösungen wurden bei zwei verschiedenen Temperaturen für einen Zeitraum von 3 bzw. 6 Monaten unter sonst identischen Bedingungen aufbewahrt. Nach 3 Monaten und gegebenenfalls auch nach 6 Monaten wurde die Aktivität der Burlulipase gemessen. Die folgende Tabelle gibt die Ergebnisse der Versuche wieder. Alle Aktivitäten sind in Prozent der bei Herstellung der Lösungen gemessenen lipolytischen Aktivität angegeben.

**Tabelle 3:**

| Talan-Gehalt | Temperatur | Beginn | 3 Monate | 6 Monate |
|---|---|---|---|---|
| 30 Gew.-% | 25 °C | 100 | 95,8 | 86,6 |
| 1 Gew.-% | 25 °C | 100 | 103,5 | 94,6 |
| 30 Gew.-% | 40 °C | 100 | 65,6 | - |
| 1 Gew.-% | 40 °C | 100 | 79,9 | - |

Wie man der Tabelle entnehmen kann ist die Burlulipase-Lösung die lediglich 1 Gew.-% 6-Desoxy-Talan enthält stabiler als die Vergleichslösung mit 30 Gew.-%. Die Verwendung von Burlulipase mit geringem 6-Desoxy-Talan-Gehalt in Arzneimittel ist daher sinnvoll.

## Patentansprüche

1. Eine wässrige Lösung umfassend Burlulipase, **dadurch gekennzeichnet, dass** sie Kalziumionen enthält.

2. Eine wässrige Lösung gemäß Anspruch 1, **dadurch gekennzeichnet, dass** sie Kalziumchlorid enthält.

3. Eine wässrige Lösung gemäß einem der Ansprüche 1 oder 2, die die Burlulipase in einer Konzentration von 0,1 bis 35 mg Protein/ml, bevorzugt in einer Konzentration von 0,5 bis 30 mg Protein/ml, ganz besonders bevorzugt in einer Konzentration von 1 bis 25 mg Protein/ml und am meisten bevorzugt in einer Konzentration von 3 bis 20 mg Protein/ml, enthält.

4. Eine wässrige Lösung gemäß einem der Ansprüche 1, 2 oder 3, die Kalziumionen in einer Konzentration von 0,1 bis 200 mmol/l, bevorzugt in einer Konzentration von 0,2 bis 100 mmol/l und ganz besonders bevorzugt in einer Konzentration von 0,5 bis 50 mmol/l enthält.

5. Eine wässrige Lösung gemäß einem der vorangehenden Ansprüche, die Kalziumchlorid in einer Konzentration von bis zu 200 mmol/l, bevorzugt in einer Konzentration von 0,01 bis 150 mmol/l, ganz besonders bevorzugt in einer Konzentration von 0,1 bis 50 mmol/l und am meisten bevorzugt in einer Konzentration von 0,5 bis 20 mmol/l, enthält.

6. Eine wässrige Lösung gemäß einem der vorangehenden Ansprüche, die einen pH-Wert von 4 bis 9, bevorzugt von 6 bis 8, ganz besonders bevorzugt von 7 bis 8 und am meisten bevorzugt von 7,3 bis 7,7 aufweist.

7. Eine wässrige Lösung gemäß einem der vorangehenden Ansprüche, die Tris(hydroxymethyl)aminomethan enthält.

8. Eine wässrige Lösung gemäß einem der vorangehenden Ansprüche, umfassend weitere Begleitstoffe ausgewählt aus 6-Desoxy-Talan, Lipide und/oder Rhamnolipide.

9. Arzneimittel umfassend eine wässrige Lösung gemäß einem der vorangehenden Ansprüche.

10. Arzneimittel gemäß Anspruch 9 zur Behandlung von exokriner Pankreasinsuffizienz.

11. Arzneimittel zur Behandlung von exokriner Pankreasinsuffizienz gemäß einem der Ansprüche 9 oder 10 zur Behandlung von Pankreasinsuffizienz bei Patienten mit Mukoviszidose.

12. Arzneimittel zur Behandlung von exokriner Pankreasinsuffizienz gemäß einem der Ansprüche 9, 10 oder 11 zur Anwendung nach sequenzieller Lagerung.

13. Arzneimittel zur Behandlung von exokriner Pankreasinsuffizienz gemäß einem der Ansprüche 9 bis 12 zur Anwendung in der Pädiatrie.

14. Eine wässrige Lösung gemäß einem der Ansprüche 1 bis 8 oder Arzneimittel gemäß des Anspruchs 9 worin die Lösung oder das Arzneimittel in einem Behälter verpackt ist, der nach dem Blow-Fill-Seal-Verfahren hergestellt wurde.

15. Verfahren zur Herstellung einer verpackten wässrigen Lösung oder eines verpackten Arzneimittels gemäß Anspruch 14, worin eine wässrige Lösung gemäß einem der Ansprüche 1 bis 8 oder ein Arzneimittel gemäß des Anspruchs 9 nach dem Blow-Fill-Seal-Verfahren verpackt wird.

## Claims

1. An aqueous solution comprising burlulipase, **characterized in that** it contains calcium ions.

2. An aqueous solution according to claim 1, **characterized in that** it contains calcium chloride.

3. An aqueous solution according to any one of claims 1 or 2, which contains the burlulipase in a concentration of 0.1 to 35 mg protein / ml, preferably in a concentration of 0.5 to 30 mg protein / ml, particularly preferably in a concentration of 1 to 25 mg protein / ml and most preferably in a concentration of 3 to 20 mg protein / ml.

4. An aqueous solution according to any one of claims 1, 2 or 3, which contains calcium ions in a concentration of 0.1 to 200 mmol / I, preferably in a concentration of 0.2 to 100 mmol / I and particularly preferably in a concentration of 0.5 to 50 mmol / I.

5. An aqueous solution according to any one of the preceding claims, which contains calcium chloride in a concentration of up to 200 mmol / I, preferably in a concentration of 0.01 to 150 mmol / I, particularly preferably in a concentration of 0.1 to 50 mmol / and most preferably in a concentration of 0.5 to 20 mmol / I.

6. An aqueous solution according to any one of the preceding claims, which has a pH value of 4 to 9, preferably of 6 to 8, particularly preferably of 7 to 8, and most preferably of 7.3 to 7.7.

7. An aqueous solution according to any one of the preceding claims which contains tris (hydroxymethyl) aminomethane.

8. An aqueous solution according to any one of the preceding claims comprising further associated material, selected from 6-deoxy talan, lipids and / or rhamnolipids.

9. A pharmaceutical product comprising an aqueous solution according to any one of the preceding claims.

10. A pharmaceutical product according to claim 9 for the treatment of exocrine pancreatic insufficiency.

11. A pharmaceutical product for the treatment of exocrine pancreatic insufficiency according to one of claims 9 or 10 for the treatment of pancreatic insufficiency in patients with cystic fibrosis.

12. A pharmaceutical product for the treatment of exocrine pancreatic insufficiency according to any one of claims 9, 10 or 11 for use after sequential storage.

13. A pharmaceutical product for the treatment of exocrine pancreatic insufficiency according to one of claims 9 to 12 for use in paediatrics.

14. An aqueous solution according to any one of claims 1 to 8 or a pharmaceutical product according to claim 9, wherein the solution or the pharmaceutical product is packaged in a container made according to the blow fill seal method.

15. A process for producing a packaged aqueous solution or packaged a pharmaceutical product according to claim 14, wherein an aqueous solution according to any one of claims 1 to 8 or a pharmaceutical product according to claim 9 is packaged according to the blow fill seal method.

## Revendications

1. Solution aqueuse comprenant de la burlulipase, **caractérisée en ce qu'**elle contient des ions de calcium.

2. Solution aqueuse selon la revendication 1, **caractérisée en ce qu'**elle contient du chlorure de calcium.

3. Solution aqueuse selon l'une des revendications 1 ou 2, contenant ladite burlulipase dans une concentration comprise entre 0,1 et 35 mg de protéine / mL, de préférence dans une concentration comprise entre 0,5 et 30 mg de protéine / mL, avec une préférence toute particulière dans une concentration comprise entre 1 et 25 mg de protéine / mL et, de la manière la plus préférée, dans une concentration comprise entre 3 et 20 mg de protéine / mL.

4. Solution aqueuse selon l'une des revendications 1, 2 ou 3, contenant lesdits ions de calcium dans une concentration comprise entre 0,1 et 200 mmol/L, de préférence dans une concentration comprise entre 0,2 et 100 mmol/L et, avec une préférence toute particulière, dans une concentration comprise entre 0,5 et 50 mmol/L.

5. Solution aqueuse selon l'une des revendications précédentes, contenant du chlorure de calcium dans une concentration pouvant aller jusqu'à 200 mmol/L, de préférence dans une concentration comprise entre 0,01 et 150 mmol/L, avec une préférence toute particulière dans une concentration comprise entre 0,1 et 50 mmol/L et, de la manière la plus préférée, dans une concentration comprise entre 0,5 et 20 mmol/L.

6. Solution aqueuse selon l'une des revendications précédentes, ayant un pH compris entre 4 et 9, de préférence compris entre 6 et 8, avec une préférence toute particulière compris entre 7 et 8 et, de la manière la plus préférée, compris entre 7,3 et 7,7.

7. Solution aqueuse selon l'une des revendications précédentes, contenant du tris-(hydroxyméthyl)aminométhane.

8. Solution aqueuse selon l'une des revendications précédentes, comprenant des substances secondaires choisies parmi le 6-désoxy-talane, des lipides et/ou des rhamnolipides.

9. Médicament, comprenant une solution aqueuse selon l'une des revendications précédentes.

10. Médicament selon la revendication 9, destiné à traiter l'insuffisance pancréatique exocrine.

11. Médicament destiné à traiter l'insuffisance pancréatique exocrine selon l'une des revendications 9 ou 10, pour le traitement de l'insuffisance pancréatique exocrine chez des patients atteints de mucoviscidose.

12. Médicament destiné à traiter l'insuffisance pancréatique exocrine selon l'une des revendications 9, 10 ou 11, pour l'utilisation suite à un stockage séquentiel.

13. Médicament destiné à traiter l'insuffisance pancréatique exocrine selon l'une des revendications 9 à 12, pour l'utilisation en soins pédiatriques.

14. Solution aqueuse selon l'une des revendications 1 à 8, ou médicament selon la revendication 9, la solution ou le médicament étant emballé(e) dans un récipient qui a été fabriqué selon le procédé de formage-remplissage-scellage.

15. Procédé destiné à fabriquer une solution aqueuse emballée ou un médicament emballé selon la revendication 14, consistant entre autres à emballer une solution aqueuse selon l'une des revendications 1 à 8 ou un médicament selon la revendication 9 en mettant en œuvre le procédé de formage-remplissage-scellage.
